# FASCICULE DE BREVET EUROPEEN

(11) **EP 1 263 784 B1**
(45) Date de publication et mention de la délivrance du brevet: **26.07.2006**
(21) Numéro de dépôt: 01911811.6
(22) Date de dépôt: 28.02.2001
(51) Int. Cl.: C07K 14/47, C12N 15/12, C12N 5/10, C07K 16/18, A61K 38/17, A61K 48/00, A61K 39/395

(54) **NOUVELLE PROTEINE ULIP/CRMP HUMAINE ET SON UTILISATION DANS LE DIAGNOSTIC ET LA THERAPIE DES CANCERS ET DES SYNDROMES NEUROLOGIQUES PARANEOPLASIQUES**
NEUES MENSCHLICHES ULIP/CRMP PROTEIN UND SEINE VERWENDUNG ZUR DIAGNOSE ZUR BEHANDLUNG VON KREBS UND VON NEUROLOGISCHEN PARANEOPLASTISCHEN SYNDROMEN
NOVEL HUMAN ULIP/CRMP PROTEIN AND USE THEREOF IN DIAGNOSIS AND TREATMENT OF CANCERS AND PARANEOPLASTIC NEUROLOGICAL SYNDROMES

(30) Priorité: 29.02.2000 FR 0002566; 18.04.2000 FR 0005005
(43) Date de publication de la demande: 11.12.2002
(73) Titulaire: INSTITUT NATIONAL DE LA SANTE ET DE LA RECHERCHE MEDICALE (INSERM), 75013 Paris (FR)
(72) Inventeur: AGUERA, Michèle, F-69300 Caluire (FR); ANTOINE, Jean-Christophe, F-42600 Chalin Le Comtal (FR); BELIN, Marie-Françoise, F-69002 Lyon (FR); HONNORAT, Jérôme, F-69500 Bron (FR); ROGEMONT, Véronique, F-69003 Lyon (FR)
(74) Mandataire: Bernasconi, Jean Raymond
(86) Numéro de dépôt international: PCT/FR2001/000589
(87) Numéro de publication internationale: WO 2001/064737

(56) Documents cités:
- FR-A- 2 759 701
- DATABASE GENEMBL [en ligne] 19 janvier 2000 (2000-01-19) YANAGI, S. ET AL.: "Rattus norvegicus mRNA for dihydropyrimidinase-related protein, complete cds." XP002157134 -& INATOME ET AL.: "Identification of CRAM, a novel unc-33 gene family protein that associates with CRMP3 and protein-tyrosine kinase(s) in the developing rat brain" J.BIOL.CHEM, vol. 275, no. 35, 1 septembre 2000 (2000-09-01), pages 27291-27302, XP002157142
- DATABASE GENEMBL [en ligne] 22 juillet 1999 (1999-07-22) HILLIER ET AL.: "au56c05.y1 Scheneider fetal brain 0004 Homo sapiens cDNA clone IMAGE:2518760 5' similar to SW:DPY3_HUMAN Q14195 dihydropirimidinase related protein-3, mRNA sequence" XP002157135
- DATABASE GENEMBL [en ligne] 22 juillet 1999 (1999-07-22) HILLIER ET AL.: "au56c05.x1 Schneider fetal brain 00004 Homo sapiens cDNA clone IMAGE:2518760 3', mRNA sequence" XP002157136
- FUKADA, M. ET AL.: "Molecular characterization of CRMP5, a novel member of the collapsin response mediator protein family" J.BIOL.CHEM, vol. 275, no. 48, 1 décembre 2000 (2000-12-01), pages 37957-37965, XP002157133
- DATABASE GENEMBL [en ligne] 21 juin 2000 (2000-06-21) HORIUCHI,M.: "Homo sapiens mRNA for hypothetical protein (ORF1)" XP002157137 -& HORIUCHI ET AL.: "Ulip6, a novel unc-33 and dihydropyrimidinase related protein highly expressed in developing rat brain" FEBS LETTERS, vol. 480, no. 2-3, 1 septembre 2000 (2000-09-01), pages 283-286, XP000961794
- DATABASE GENEMBL [en ligne] 6 juillet 2000 (2000-07-06) YU, Z. ET AL.: "Homo sapiens collapsin response mediator protein-5 (CRMP5) mRNA; complete cds." XP002157138

## Description

L'invention concerne une nouvelle protéine ULIP/CRMP humaine et son utilisation dans le diagnostic et la thérapie des cancers et des syndromes neurologiques paranéoplasiques.

Les syndromes neurologiques paranéoplasiques (SNP) surviennent à l'occasion d'un cancer, souvent avant sa découverte et ne sont liés ni à la prolifération tumorale elle-même (envahissement direct, métastases) ni à la thérapie. Leur fréquence est globalement estimée à environ 1 % des cancers. Plusieurs tableaux cliniques ont été depuis longtemps individualisés (encéphalomyélite, neuropathie sensitive de Denny Brown, atrophie cérébelleuse, encéphalite limbique, opsoclonus, ...) correspondant en fait à l'atteinte soit élective soit préférentielle de certains groupes de neurones. La fréquence des cellules inflammatoires au voisinage des lésions avait fait évoquer depuis de nombreuses années la possibilité d'un processus auto-immun ou viral. La mise en évidence, plus récente, d'auto-anticorps dans le sérum et le liquide céphalo-rachidien (LCR) de patients souffrant de SNP, spécifiques du type de tumeur et du type de neurones qui dégénèrent, a relancé l'hypothèse d'une participation de l'auto-immunité dans la genèse de cette pathologie (Graus et al., 1985 ; Greenlee et al., 1983).

Outre la présence d'un titre élevé de ces anticorps dans le sang et le LCR des patients, il existe plusieurs arguments suggérant que les SNP relèvent de mécanismes auto-immuns. Ainsi, les antigènes reconnus dans le système nerveux central sont aussi présents dans les tumeurs des patients (Anderson et al., 1987). Au sein du tissu tumoral, on retrouve des anticorps spécifiquement dirigés contre ces antigènes ainsi que des lymphocytes B et T (Hetzel et al., 1990).

Ces données suggèrent que le processus auto-immun serait déclenché par l'expression d'antigènes tumoraux. Un processus d'immunité croisée provoquerait les lésions du système nerveux central. D'autres arguments indiquent en outre que les lésions cérébrales résultent de la réponse auto-immune. Ainsi, dans le cerveau des patients, le titre des anticorps spécifiques est supérieur à celui du sérum et du LCR (Dalmau et al., 1991). De plus, dans le cas des encéphalomyélites associées aux anticorps anti-Hu, il existe une réaction lymphocytaire intense, composée de cellules B et T, située à proximité de neurones en voie de destruction (Dalmau et al., 1991 ; Graus et al., 1990).

Plusieurs types d'auto-anticorps permettant des regroupements syndromiques précis en fonction de critères immunologiques, neurologiques et carcinologiques ont été décrits.

Ainsi, les anticorps anti-Yo sont retrouvés dans le sérum et le LCR de femmes présentant une atrophie cérébelleuse paranéoplasique et un cancer gynécologique (ovaire, sein ou utérus) (Greenlee et al., 1983 ; Jaeckle et al., 1985).

Ces anticorps reconnaissent deux protéines cytoplasmiques de 34 et 62 kDa spécifiques des cellules de Purkinje du cervelet.

Les anticorps anti-Ri sont retrouvés dans le sérum et le LCR de patients (principalement des femmes) présentant un opso-myoclonus, un syndrome cérébelleux et un cancer du sein. Ces anticorps reconnaissent deux protéines de 50 et 80 kDa spécifiques des neurones du système nerveux central (Luque et al., 1991).

Les anticorps anti-Hu sont les plus fréquemment rencontrés au cours des SNP. Ils sont retrouvés dans le sérum et le LCR de patients présentant un syndrome de Denny-Brown ou une encéphalomyélonévrite et un cancer du poumon à petites cellules (Graus et al., 1985 ; Dalmau et al., 1992). Ces auto-anticorps reconnaissent plusieurs protéines de 37 à 45 kDa exprimées spécifiquement par l'ensemble des neurones du système nerveux.

Un autre type d'auto-anticorps a été identifié chez des patients présentant un SNP : les anticorps anti-CV2 (Antoine et al., 1993 ; Honnorat et al., 1996). Ces derniers sont atypiques, en ce sens que la cible antigénique reconnue à l'âge adulte est essentiellement non neuronale, alors que l'analyse du cerveau *post-mortem* de quatre patients permet d'objectiver une perte neuronale, une gliose et un processus inflammatoire caractéristique des SNP.

L'originalité de la découverte de ces auto-anticorps réside, d'une part, dans leur mise en évidence. Ces derniers avaient échappé à l'ensemble des investigations habituelles qui consistaient à révéler les antigènes reconnus par immunohistochimie sur du cerveau *post-mortem.* L'antigène reconnu est en effet soluble et disparaît du cerveau *post-mortem* dans la plupart des conditions de fixation. Seule une fixation du tissu *post-mortem* humain par immersion dans le paraformaldéhyde ou *in situ* par perfusion de paraformaldéhyde chez l'animal, a permis de révéler la présence de ces anticorps dans le LCR ou le sérum des patients atteints de SNP (Antoine et al., 1993 ; Honnorat et al., 1996).

Les auto-anticorps anti-CV2 présents dans les sérums de patients atteints de syndrome neurologique paranéoplasique (SNP) ont été définis par leur capacité à reconnaître, par immunohistochimie indirecte, un antigène cytoplasmique exprimé spécifiquement, dans le cerveau de rat adulte, par une sous-population d'oligodendrocytes du tronc cérébral, de la moelle et du cervelet.

L'originalité de ces auto-anticorps réside, d'autre part, dans leur intérêt diagnostique. Leur présence dans le sérum ou le LCR de patients a valeur diagnostique puisqu'elle permet de préciser l'origine paranéoplasique d'un syndrome neurologique. La découverte de ces anticorps lorsqu'elle précède celle du cancer, oriente la recherche de celui-ci et permet sa découverte. Tel a été le cas pour six patients sur 19 présentant des anticorps anti-CV2. Les troubles cliniques étaient différents suivant les patients, certains présentaient un tableau d'encéphalite limbique, d'autres une encéphalomyélonévrite et d'autres un syndrome de Lambert-Eaton. Néanmoins, dans plus de 60 % des cas, le syndrome cérébelleux était prédominant. La tumeur la plus fréquemment associée était le cancer du poumon. à petites cellules (60 % des cas).

Des expériences sur des cerveaux de rats nouveaux-nés ont montré que ces anticorps anti-CV2 réagissaient avec une protéine de 66 kDa (Honnorat et al., 1996).

Cet antigène se situe dans le cerveau adulte dans une sous-population d'oligodendrocytes ou dans des cellules qui gardent des capacités de différenciation dans le cerveau adulte (bulbe olfactif, gyrus denté). L'antigène reconnu jouerait un rôle dans la survie neuronale, via des interactions Neurone/Oligodendrocyte, comme le suggère la perte des neurones observée dans le cerveau *post-mortem* de patients atteints de SNP.

Son expression très restreinte à l'âge adulte contraste avec une expression très forte et transitoire dans le système nerveux central et périphérique en développement, suggérant le rôle probable de cet antigène dans le développement du système nerveux.

Dans la demande WO 98/37192 et l'article de Honnorat et al de 1999, l'antigène cible des auto-anticorps anti-CV2, correspondant à une protéine désignée par «POP-66» pour « paraneoplastic oligodendrocyte protein 66 kDa », a été identifiée comme étant la forme humaine de la protéine ULIP-4. Les protéines ULIP (pour "Unc-33 like phosphoprotein") sont impliquées dans le contrôle du développement neuronal et le transport axonal (Byk et al, 1996). Quatre membres de cette famille avaient été identifiés par trois équipes différentes (Byk et al, 1998, Wang et Strittmatter, 1996 ; et Hamajima et al, 1996). La recherche extensive d'éventuels autres membres de cette famille n'avait pas abouti.

Les auteurs de la présente invention ont alors été confrontés à de nouveaux résultats, qui n'étaient pas cohérents avec l'identification établie par la demande WO 98/37192 : Alors que tous les sérums anti-CV2 testés sur des cellules Hela reconnaissaient indiscutablement la protéine recombinante ULIP 4 en immunohistochimie, seuls 20% de ces sérums reconnaissaient la protéine ULIP 4 par Western Blot sur ces mêmes extraits de cellules. Or tous les sérums anti-CV2 testés reconnaissaient par ailleurs par Western Blot la même protéine de 66 kDa après immunoprécipitation, sur des extraits de cerveaux. En outre, l'ARNm d'ULlP4 n'était que très faiblement exprimé dans les oligodendrocytes en hybridation *in situ.* A partir de ces données, les auteurs de la présente invention ont supposé qu'il pouvait exister un nouveau membre de la famille des ULIP non identifié à ce jour, fortement exprimé par les oligodendrocytes et reconnu en Western Blot par tous les sérums anti-CV2.

Les auteurs de la présente invention ont maintenant mis en évidence que, contrairement à ce que proposait la demande WO 96/37192, POP-66, l'antigène majeur cible des auto-anticorps anti-CV2, n'était pas la protéine ULIP 4 mais une autre protéine. Ils sont parvenus à caractériser celle-ci. Il s'agit d'une nouvelle protéine humaine de la famille des ULIP, désignée ULIP6.

ULIP 6 comprend dans sa partie C-terminale un épitope majeur reconnu en Western Blot par les anticorps anti-CV2.

La présente invention a donc pour objet un polypeptide purifié ULIP 6, comprenant la séquence d'acides aminés SEQ ID n° 2.

Est en outre compris dans la présente invention un fragment épitopique du polypeptide mentionné ci-dessus, comprenant la séquence SEQ ID n°4. Plus particulièrement l'invention a pour objet le peptide purifié de séquence SEQ ID n°4.

La présente invention a également pour objet un acide nucléique isolé codant pour le polypeptide ULIP6 tel que défini ci-dessus, de préférence comprenant la séquence nucléotidique SEQ ID n° 1. La séquence SEQ ID n°1 présente une région 5' non codante (nucléotides 1 à 162), un cadre de lecture ouvert (nucléotides 163 à 1854) et une région 3' non codante (nucléotides 1855 à 3074).

La présente invention a en outre pour objet un acide nucléique isolé comprenant la séquence nucléotidique SEQ ID n°3, qui correspond à la région non codante en 3' de la séquence codante humaine SEQ ID n°1. Cette séquence non codante de même que la partie 5' non codante (nucléotides 1 à 162 à SEQ ID n° 1) peuvent notamment servir à la préparation de sondes spécifiques.

Le polypeptide de la présente invention peut être synthétisé par toutes les méthodes bien connues de l'homme du métier. Le polypeptide de l'invention peut par exemple être synthétisé par les techniques de la chimie de syrithèse, telles que la synthèse de type Merrifield qui est avantageuse pour des raisons de pureté, de spécificité antigénique, d'absence de produits secondaires non désirés et pour sa facilité de production.

Une protéine ULIP6 recombinante peut également être produite par un procédé, dans lequel un vecteur contenant un acide nucléique comprenant la séquence SEQ ID n° 1 est transféré dans une cellule hôte qui est mise en culture dans des conditions permettant l'expression du polypeptide correspondant.

La protéine produite peut ensuite être récupérée et purifiée.

Les procédés de purification utilisés sont connus de l'homme du métier. Le polypeptide recombinant obtenu peut être purifié à partir de lysats et extraits cellulaires, du surnageant du milieu de culture, par des méthodes utilisées individuellement ou en combinaison, telles que le fractionnement, les méthodes de chromatographie, les techniques d'immunoaffinité à l'aide d'anticorps mono- ou polyclonaux spécifiques, etc.

La séquence d'acide nucléique d'intérêt, codant pour le polypeptide ULIP6, peut être insérée dans un vecteur d'expression, dans lequel elle est liée de manière opérante à des éléments permettant la régulation de son expression, tels que notamment des promoteurs, activateurs et/ou terminateurs de transcription.

Les signaux contrôlant l'expression des séquences nucléotidiques (promoteurs, activateurs, séquences de terminaison...) sont choisis en fonction de l'hôte cellulaire utilisé. A cet effet, les séquences nucléotidiques selon l'invention peuvent être insérées dans des vecteurs à réplication autonome au sein de l'hôte choisi, ou des vecteurs intégratifs de l'hôte choisi. De tels vecteurs seront préparés selon les méthodes couramment utilisées par l'homme du métier, et les clones en résultant peuvent être introduits dans un hôte approprié par des méthodes standard, telles que par exemple l'électroporation ou la précipitation au phosphate de calcium.

Les vecteurs de clonage et/ou d'expression tels que décrits ci-dessus, contenant une séquence nucléotidique définie selon l'invention font également partie de la présente invention.

L'invention vise en outre les cellules hôtes transfectées, de manière transitoire ou stable, par ces vecteurs d'expression. Ces cellules peuvent être obtenues par l'introduction dans des cellules hôtes d'une séquence nucléotidique insérée dans un vecteur tel que défini ci-dessus, puis la mise en culture desdites cellules dans des conditions permettant la réplication et/ou l'expression de la séquence nucléotidique transfectée.

L'hôte cellulaire peut être choisi parmi des systèmes procaryotes, comme les bactéries, ou eucaryotes, comme par exemple les levures, cellules d'insectes, CHO (cellules d'ovaires de hamster chinois) ou tout autre système avantageusement disponible. Un hôte cellulaire préféré pour l'expression des protéines de l'invention est constitué par la bactérie *E. coli.*

Les séquences nucléotidiques de l'invention peuvent être d'origine artificielle ou non. Il peut s'agir de séquences d'ADN ou d'ARN, obtenues par criblage de banques de séquences au moyen de sondes élaborées sur la base de la séquence SEQ ID n° 1 ou 3. De telles banques peuvent être préparées par des techniques classiques de biologie moléculaire, connues de l'homme de l'art.

Les séquences nucléotidiques selon l'invention peuvent également être préparées par synthèse chimique, ou encore par des méthodes mixtes incluant la modification chimique ou enzymatique de séquences obtenues par criblage des banques.

Cet acide nucléique permet la réalisation de sondes nucléotidiques, capables de s'hybrider fortement et spécifiquement avec une séquence d'acide nucléique, d'un ADN génomique ou d'un ARN messager, codant pour un polypeptide selon l'invention ou un fragment biologiquement actif de celui-ci. Les conditions d'hybridation appropriées correspondent aux conditions de température et de force ionique usuellement utilisées par l'homme du métier (Sambrook et al., 1989), de préférence à des conditions de température comprises entre (Tₘ moins 5°C) et (Tₘ moins 30°C) et de préférence encore, à des conditions de température comprises entre (Tₘ moins 5°C) et (Tₘ moins 10°C) (forte stringence), Tm étant la température théorique de fusion, définie comme étant la température à laquelle 50 % des brins appariés se séparent. De telles sondes font également partie de l'invention. Elles peuvent être utilisées comme outil de diagnostic *in vitro* pour la détection, par des expériences d'hybridation, de transcrits spécifiques des polypeptides de l'invention dans des échantillons biologiques ou pour la mise en évidence de synthèses aberrantes ou d'anomalies génétiques résultant d'un polymorphisme, de mutations ou d'un mauvais épissage.

Les sondes de l'invention comportent au minimum 10 nucléotides, et au maximum comportent la totalité d'une séquence nucléotidique SEQ ID n° 1 ou 3, ou de son brin complémentaire.

L'acide nucléique de l'invention peut également être utilisé pour réaliser des amorces oligonucléotidiques , qui hybrident dans des conditions de forte stringence à la séquence SEQ ID n° 1 ou 3.

Ces amorces oligonucléotidiques sens et/ou antisens peuvent être utiles pour des réactions de séquençage ou d'amplification spécifique selon la technique dite de PCR (réaction de polymérisation en chaîne) ou toute autre variante de celle-ci.

Préférentiellement, les sondes ou amorces de l'invention sont marquées, préalablement à leur utilisation. Pour cela, plusieurs techniques sont à la portée de l'homme du métier comme par exemple le marquage fluorescent, radioactif, chimioluminescent ou enzymatique.

Les méthodes de diagnostic *in vitro* dans lesquelles ces sondes nucléotidiques sont mises en oeuvre pour la détection de synthèses aberrantes ou d'anomalies génétiques, telles que la perte d'hétérozygotie et le réarrangement génétique, au niveau des séquences nucléiques codant pour un polypeptide ULIP6 selon l'invention sont incluses dans,la présente invention. Un tel type de méthode comprend :
- la mise en contact d'une sonde nucléotidique de l'invention avec un échantillon biologique dans des conditions permettant la formation d'un complexe d'hybridation entre ladite sonde et la susdite séquence nucléotidique, éventuellement après une étape préalable d'amplification de la susdite séquence nucléotidique ;
- la détection du complexe d'hybridation éventuellement formé;
- éventuellement le séquençage de la séquence nucléotidique formant le complexe d'hybridation avec la sonde de l'invention.
   Les sondes de l'invention sont en outre avantageusement utilisables pour la détection d'anomalies chromosomiques.
   Les séquences nucléotidiques selon l'invention ont par ailleurs des utilisations dans le domaine thérapeutique, pour la réalisation de séquences antisens, capables de s'hybrider spécifiquement avec une séquence d'acide nucléique, y compris un ARN messager, utilisables en thérapie génique. L'invention a ainsi pour objet des séquences antisens capables d'inhiber, au moins partiellement, la production d'un polypeptide selon l'invention, tel que défini précédemment.
   Elles sont plus particulièrement utiles dans le traitement des désordres du système nerveux central et périphérique et de la vision, notamment dans le traitement des syndromes neurologiques paranéoplasiques, ainsi que dans le traitement anticancéreux, notamment des tumeurs associées à des syndromes neurologiques paranéoplasiques.
   L'exploitation des protéines ULIP, et en particulier ULIP6, ainsi que des anticorps dirigés contre ces protéines, est prometteuse dans divers domaines.
   Ainsi, la détection de l'auto-anticorps anti-CV2 par immunofluorescence sur cerveau animal fixé est utilisée actuellement comme test diagnostic.
   La production de protéine recombinante ULIP6, selon l'invention permet la fabrication d'un test (de type Elisa ou Western Blot) rapide et fiable, pour détecter les anticorps anti-CV2.
   De tels tests existent déjà pour les anticorps anti-Hu, anti-Yo et anti-Ri. Le test pour détecter les anti-CV2 dans le sérum des patients pourrait être prescrit en cas de suspicion de syndrome neurologique paranéoplasique et inclurait par conséquent les anticorps anti-CV2 au même titre que les autres anticorps identifiés dans les SNP tels que précédemment cités.
   L'invention vise donc également une méthode pour le diagnostic des syndromes neurologiques paranéoplasiques et/ou pour le diagnostic précoce de la formation de tumeurs d'origine cancéreuse, caractérisée en ce que l'on met en évidence dans un échantillon biologique (tel que sang, sérum, LCR, etc) prélevé chez un individu des auto-anticorps dirigés contre une protéine ULIP6 par
- la mise en contact un échantillon biologique prélevé chez un individu avec un polypeptide purifié ULIP6 éventuellement fixé sur un support dans des conditions permettant la formation de complexes immunologiques spécifiques entre ledit polypeptide et les auto-anticorps éventuellement présent dans l'échantillon biologique, et
- la détection des complexes immunologiques spécifiques éventuellement formés.

L'invention a donc pour objet une composition utile pour le diagnostic des syndromes neurologiques paranéoplasiques et/ou pour le diagnostic précoce de la formation des tumeurs, caractérisée en ce qu'elle comprend un polypeptide ULIP6 ou un fragment épitopique dudit polypeptide.

De manière avantageuse, on peut utiliser à la place du polypeptide complet la partie C-términale comprenant l'épitope dominant (par exemple le fragment allant de l'acide aminé n° 475 à l'acide aminé 564). On peut en particulier utiliser un fragment épitopique du polypeptide ULIP 6, comprenant la séquence SEQ ID n°4. Le peptide de séquence SEQ ID n°4 a ainsi permis la production d'anticorps très spécifiques d'ULIP6.

L'invention a également pour objet un kit pour le diagnostic des syndromes neurologiques paranéoplasiques et pour le diagnostic précoce de la formation des tumeurs à partir d'un prélèvement biologique comprenant :
- au moins un polypeptide purifié ULIP6, éventuellement fixé sur un support,
- des moyens de révélation de la formation de complexes antigène/anticorps spécifiques entre un auto-anticorps anti-ULIP6 et ledit polypeptide purifié ULIP6, dérivé ou fragment polypeptidique et/ou des moyens de quantification de ces complexes.

L'invention a également pour objet les anticorps mono- ou polyclonaux ou leurs fragments, anticorps chimériques ou immunoconjugués, obtenus à partir d'un peptide ou polypeptide purifié ULIP comprenant une séquence d'acides aminés SEQ ID n° 2 ou n°4 et leur utilisation, pour la purification ou la détection d'une protéine ULIP dans un échantillon biologique.

Des anticorps polyclonaux peuvent être obtenus à partir du sérum d'un animal immunisé contre la protéine, produite par exemple par recombinaison génétique suivant la méthode décrite ci-dessus, selon les modes opératoires usuels.

Les anticorps monoclonaux peuvent être obtenus selon la méthode classique de culture d'hybridomes décrite par Köhler et Milstein.

Les anticorps peuvent être des anticorps chimériques, des anticorps humanisés, des fragments Fab et F(ab')2. Ils peuvent également se présenter sous forme d'immunoconjugués ou d'anticorps marqués.

L'invention porte également sur l'utilisation d'anticorps dirigés contre la protéine ULIP6 pour la mise en évidence d'une protéine ULIP6 dans des néoplasmes, et des syndromes neurologiques paranéoplasiques à des fins de diagnostic.

De manière préférentielle, l'invention porte sur l'utilisation d'anticorps monoclonaux obtenus à partir du sérum polyclonal anti-CV2 de patients par immortalisation de lymphocytes, selon les techniques usuelles connues de l'homme du métier.

Ainsi, les anticorps dirigés contre une protéine de la famille ULIP sont utiles pour détecter une expression anormale de protéine ULIP chez des patients présentant des syndromes neurologiques, chez qui aucun cancer n'a été diagnostiqué par les méthodes classiques. Cette expression anormale de protéine ULIP6 pourra être corrélée à l'existence d'un cancer qui n'avait pas été repéré. Ainsi, les anticorps dirigés contre la protéine ULIP6, sont utiles pour le diagnostic précoce d'un cancer.

Des anticorps humains ou non, obtenus chez des patients, ou après immunisation avec l'ensemble ou une partie de la protéine ULIP6 tels que définis précédemment, peuvent également être marqués de manière détectable par exemple par association à un élément radioactif, et être injectés à un individu. Par des procédés d'imagerie bien connus de l'homme du métier, ils peuvent permettre de détecter ou diagnostiquer un tumeur cancéreuse après réaction antigénique de ces anticorps avec les cellules de la tumeur.

L'invention a donc également pour objet une méthode de détection ou de diagnostic d'une tumeur cancéreuse comprenant l'administration à un patient d'un anticorps tel que défini précédemment marqué de manière détectable et la visualisation par imagerie du site de fixation de cet anticorps.

L'invention a également pour objet une composition pharmaceutique comprenant au moins un agent thérapeutique choisi parmi une protéine purifiée ULIP6, ou un acide nucléique codant pour ladite protéine, une séquence anti-sens capable de s'hybrider spécifiquement avec une séquence nucléotidique SEQ ID n°1 ou n°3, ou un anticorps dirigé contre ladite protéine, associée à un véhicule pharmaceutiquement acceptable.

L'invention comprend de manière préférentielle des compositions pharmaceutiques comprenant comme principe actif un polypeptide ULIP6 purifié, préférentiellement sous forme soluble, associé à un véhicule pharmaceutiquement acceptable.

De telles compositions offrent une nouvelle approche pour traiter les désordres du système nerveux central et périphérique et de la vision, et notamment les syndromes neurologiques paranéoplasiques. Par ailleurs, elles sont utiles pour traiter les désordres neurologiques liés à une perte neuronale et/ou une sous-expression de la protéine ULIP6 dans le système nerveux.

Ainsi, ULIP6 révèle aussi un intérêt dans des pathologies neurodégénératives telles que les atrophies multisystémiques qui sont des affections similaires à celles des SNP et pour lesquelles une anomalie d'une sous-population oligodendrocytaire a été détectée (Papp et al., 1992).

Les compositions selon l'invention sont par ailleurs utiles en thérapie anticancéreuse.

Les anticorps dirigés contre la protéine ULIP6 peuvent être associés à des agents antinéoplasiques, permettant ainsi le ciblage des médicaments vers les cellules tumorales.

Ils peuvent en outre être associés à un groupe chimique hydrophile choisi de manière à passer ou non la barrière hématoencéphalique, selon le type de tumeur.

La protéine ULIP6 ainsi que les séquences nucléotidiques décrites plus haut, et les séquences ou oligonucléotides anti-sens, peuvent être utiles dans la thérapie de tout type de cancer dans lequel un gène codant pour la protéine ULIP6 est impliqué. Parmi des exemples de cancers, on peut citer les tumeurs périphériques, telles que le cancer du poumon à petites cellules, le thymome, le cancer du sein et de l'ovaire, ainsi que les tumeurs cérébrales, de préférence les tumeurs cérébrales primitives d'origine gliale. L'expression d'ULIP6 dans les cellules non prolifératives du cerveau normal, son absence dans des tissus normaux tels que poumon ou thymus par exemple, sa réexpression différentielle lors de la tumorigénèse de ces tissus et la modulation de son expression dans une lignée tumorale au cours de la différenciation suggèrent à cet égard que ULIP 6 pourrait être un gène suppresseur de tumeur.

On peut également utiliser un composé ou un mélange de composés d'origine synthétique ou naturelle qui inhibe l'action de l'ULIP 6.

Alternativement, une stimulation de l'ULIP 6 peut être recherchée. On peut alors utiliser un composé ou un mélange de composés d'origine synthétique ou naturelle qui active l'expression ou l'action de la protéine ULIP 6.

Ces composés activateurs ou inhibiteurs peuvent être inclus dans des compositions pharmaceutiques.

Préférentiellement, les compositions pharmaceutiques selon l'invention peuvent être administrées par voie systémique, de préférence par voie intraveineuse, par voie intramusculaire, intradermique ou par voie orale.

Leurs modes d'administration, posologies et formes galéniques optimaux peuvent être déterminés selon les critères généralement pris en compte dans l'établissement d'un traitement thérapeutique adapté à un patient comme par exemple l'âge ou le poids corporel du patient, la gravité de son état général, la tolérance au traitement et les effets secondaires constatés, etc.

L'invention comprend également l'utilisation d'une protéine purifiée ULIP6, un acide nucléique codant pour ladite protéine ou appartenant aux régions non codantes du gène de l'ULIP6, une séquence anti-sens capable de s'hybrider spécifiquement avec une séquence nucléotidique SEQ ID n°1 ou n°3, ou un anticorps dirigé contre ladite protéine, associée à un véhicule pharmaceutiquement acceptable, pour la fabrication d'un médicament destiné à traiter les maladies neurodégénératives et les néoplasmes.

L'invention a enfin pour objet une méthode de traitement des maladies neurodégénératives et des néoplasmes comprenant l'administration à un sujet nécessitant un tel traitement d'une quantité thérapeutiquement efficace d'une composition pharmaceutique telle que définie précédemment.

Les exemples et les figures dont les légendes sont présentées ci-après sont donnés à titre illustratif.

### LEGENDE DES FIGURES

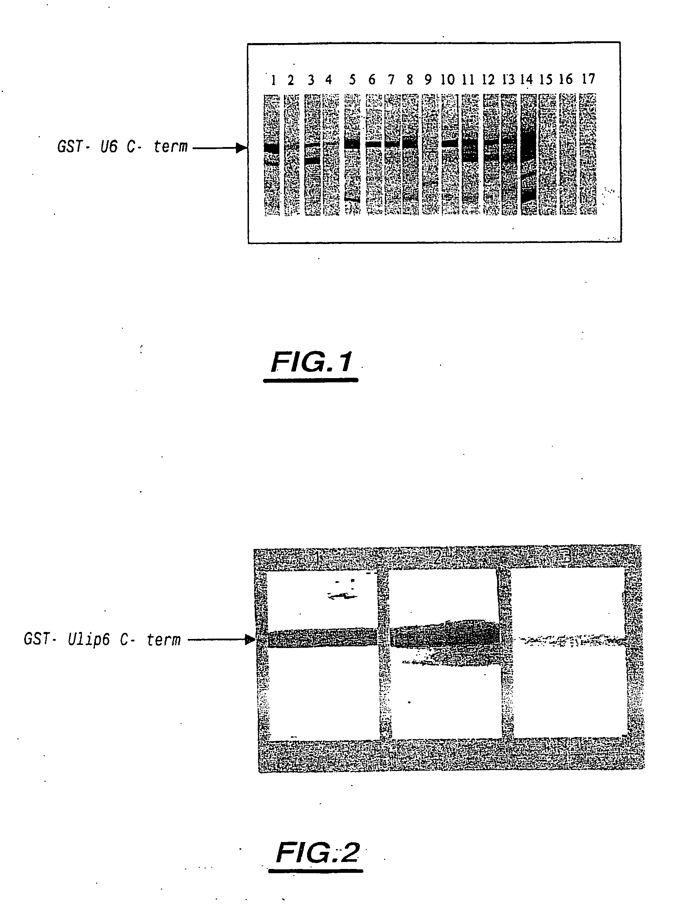

- La figure 1 représente un Western Blot réalisé à partir d'extraits protéiques d'*E. coli* exprimant la protéine de fusion GST-ULIP6 C-term. Ces extraits ont été séparés par SDS-PAGE 12,5 %, transférés sur membrane de PVDF et incubés avec des sérums humains. Pistes 1 à 14 : sérums anti-CV2 ; pistes 15 à 17 : sérums contrôles.
- La figure 2 représente un Western Blot réalisé à partir de protéines de fusion GST-ULIP6 C-term après purification sur billes d'agarose-glutathione. La protéine est reconnue par deux sérums anti-CV2, (piste 1 : sérum 94-822, piste 2 : sérum 95-590) mais pas par un sérum contrôle (ligne 3).

### Exemple 1 : Identification de la cible des anticorps anti-CV2

Après expression des protéines recombinantes de la famille Ulip dans des cellules HeLa, les auteurs de l'invention ont pu montrer que les sérums anti-CV2 alors en leur possession reconnaissaient tous Ulip4 en immunohistochimie. Ce résultat suggérait que Ulip4 pouvait être l'antigène majeur reconnu par les sérums anti-CV2 (Honnorat et al, 1999). Par contre, lorsque une cohorte plus importante de sérums a été testée en Western blot sur la protéine Ulip4 exprimée dans *E. coli,* ils se sont aperçus que si plusieurs sérums anti-CV2 reconnaissaient indiscutablement la protéine recombinante Ulip4, certains ne la reconnaissaient pas, alors que tous les sérums anti-CV2 reconnaissent par Western blot une même protéine de 66kDa après immunoprécipitation d'extraits protéiques de cerveaux (Honnorat et al, 1996). De plus, par hybridation *in situ,* les oligodendrocytes n'exprimaient pas l'ARN messager de Ulip4. Les auteurs de l'invention ont alors émis l'hypothèse de l'existence d'une autre protéine homologue aux protéines Ulip, non encore décrite et exprimée par les oligodendrocytes.

Pour rechercher cette protéine, un sérum anti-CV2, ne reconnaissant pas Ulip4 recombinante par Western blot a été utilisé pour cribler une banque d'expression. Une banque d'ADNc de moëlle épinière humaine, lieu d'expression maximale de l'antigène CV2 chez l'adulte, clonés dans le phage lambda gt11 a été choisie (Clontech, Palo Alto, USA). Les phages ont été criblés à une densité de 2x10⁴ pfu par boites de 150 mm de diamètres. Après incubation 3 heures 30 à 42°C, les boites étaient recouverte d'une membrane de nitrocellulose incubée dans l'IPTG (10 mmolaires) et réincubées 3 heures à 37°C. Les membranes étaient ensuite saturées dans du PBS-Tween-lait écrémé, puis incubées une nuit avec le sérum dilué au 1/100. Les membranes étaient ensuite lavées en PBS-Tween puis incubées avec un anti-sérum anti-immunoglobuline humaine marqué à la peroxidase. Après lavage, les membranes étaient révélées par la méthode de diaminobenzidine. Les clones donnant un signal positif ont été purifiés par repiquages successifs jusqu'a obtenir 100% de clones positifs. Quatre ADNc de 1400 à 1700 paires de bases et codant pour la partie C-terminale d'une même protéine ont été identifiés. Le clone présentant la plus grande séquence codante (clone 97) contenait 1490 paires de bases (nucléotides 1585 à 3074 de la séquence ID n°1) avec un cadre ouvert de lecture de 270 nucléotides codant pour un polypeptide de 90 acides aminés (acides aminés 475 à 564 de la séquence ID n°2). Après recherche d'homologie dans des banques de données, il a été constaté que ce polypeptide (nommé dans la suite de l'étude Ulip6 C-Term) présentait une homologie de 35% avec la partie C-terminale de chacun des membres déjà connu de la famille Ulip et qu'il n'existait aucune homologie avec d'autres familles de protéines.

### Exemple 2 : Production de la protéine recombinante GST-ULIP6 C-Term

Pour confirmer que ce polypeptide était bien l'antigène reconnu par les sérums anti-CV2, la phase codante du clone 97 a été clonée dans un vecteur d'expression bactérien, pGex 2T (Pharmacia Amersham Biotech, Suède). Ce vecteur permet l'expression, chez E.coli, de la protéine d'intérêt en fusion avec la glutathione-S-transferase (GST, 26kDa). Par Western-blot, 16 des 18 sérums anti-CV2 testés reconnaissaient la protéine de fusion GST-Ulip6-CTerm dans un extrait protéique bactérien, soit 89 % de positifs (Figure 1). Il est à noter que le polypeptide Ulip6 C-Term a un poids moléculaire de 10 kDa ce qui représente environ 15 % d'une protéine de 66 kDa. Aucun de ces sérums ne reconnaissait la GST seule. 100 sérums contrôles ont également été testés. Aucun ne présentait de réactivité vis à vis de la protéine de fusion GST-Ulip6 C-Term. Dans le but de disposer du test le plus spécifique possible, les auteurs de l'invention ont également testé les sérums anti-CV2 sur la protéine de fusion GST-Ulip6 C-Term purifiée sur billes d'agarose-glutathione. La figure 2 montre un exemple des résultats obtenus en Western blot.

### Exemple 3 : Northern-blot sur ARN de moelle épinière humaine

Pour déterminer la taille du transcript du gène Ulip6, une analyse par. Northern blot a été réalisée sur des ARN polyA+ purifiés extraits de moelle épinière humaine (Clontech, Palo Alto, USA). La sonde correspondait à la totalité du clone 97 marqué au dCTP alpha ³²P. Les ARN ont été séparés sur gel d'électrophorèse agarose 1,2% formaldéhyde et transférés sur membrane de nylon. Après préhybridation avec la solution d'hybridation rapide (Clontech, Palo Alto, USA) la membrane était incubée une heure avec la sonde. Après lavage, la membrane était exposée sur un film pendant une nuit à -80°C. Une seule bande correspondant à un transcrit de 5 kb a été révélée.

### Exemple 4 : Hybridation in situ sur moelle épinière

Pour vérifier la présence de l'ARN messager de Ulip6 dans les oligodendrocytes, une analyse par hybridation *in situ* a été réalisée sur coupe frontale de moelle. La sonde utilisée était une sonde ARN froide obtenue par transcription du clone 97 sous cloné dans pBluescript SK (Stratagène) et marquée à la digoxigénine. Une sonde sens était utilisée comme contrôle négatif. Un marquage spécifique des oligodendrocytes a pu être observé.

### Exemple 5 : Production d'un anticorps de lapin anti-Ulip6

Des anticorps polyclonaux ont été produits par immunisation de lapins contre un peptide spécifique de la protéine Ulip6 (peptide Pep Ulip6="KEMGTPLADTPTRPVTRHGG" de séquence SEQ ID n°4 , correspondant au fragment d'acides aminés 505 à 524 sur SEQ ID n°2). Le peptide a été synthétisé sur un appareil de synthèse peptidique (432A Peptide Synthesizer SYNERGY, Applied Biosystems), par la société COVALAB (Lyon, France). La pureté des échantillons a été contrôlée par HPLC et spectrométrie de masse. Un milligramme de peptide couplé à l'hémocyanine et à de l'adjuvant complet de Freund a été injecté aux lapins (COVALAB, Lyon, France). Toutes les 3 semaines, une nouvelle injection de 0,5 mg a été réalisée. La production d'anticorps et leur spécificité ont été analysées par wesiern-blots et immunohistochimie, en utilisant comme contrôle les sérums pré-immuns.

Les anticorps obtenus reconnaissaient la protéine GST-Ulip6 C-Term par western-blot, une protéine de 66 kDa sur extraits de cerveau et marquaient spécifiquement des oligodèndrocytes par immunohistochimie sur coupes de moelle de rats.

### Exemple 6 : Recherche de la séquence complète Ulip6

Pour obtenir un ADNc complet de Ulip6, la banque d'ADNc de moëlle épinière humaine, clonés dans le phage lambda gt11 (Clontech, Palo Alto, USA) a été criblée avec une sonde radioactive. Cette sonde obtenue par PCR était marquée au dCTP alpha ³²P, et correspondait aux nucléotides 1585 à 1854 de la séquence ID N°1. Les phages ont été criblés à une densité de 2x10⁴ pfu par boites de 150 mm de diamètres. Après incubation 6 heures à 37°C, une réplique était obtenue sur une membrane de nylon. Cette membrane était traitée pour dénaturation des phages; puis l'ADN était fixé une nuit à 42°C. Après préhybridation avec la solution d'hybridation rapide (Clontech, Palo Alto, USA) la membrane était incubée une heure avec la sonde radioactive. Après lavage, la membrane était exposée sur un film pendant une nuit à -80°C. des clones donnant un signal positif ont été purifiés par repiquages successifs jusqu'a obtenir 100% de clones positifs. Le plus grand ADNc obtenu comprenait 3074 nucléotides (SEQ ID n° 1) et comprenait un cadre ouvert de lecture de 1692 nucléotides (nucléotides n° 163 à 1854 de la SEQ ID n° 1). Il codait pour une protéine de 564 acides aminés (SEQ ID n° 2), dont la partie C-terminale était strictement identique au polypeptide ULIP6 C-term (acides aminés 475 à 564 sur SEQ ID n° 2). Après alignement de la protéine obtenue avec les quatre protéines ULIP/CRIUP humaines connues, une homologie de 50 % était observée.

### BIBLIOGRAPHIE

- Anderson et al., CRC Crit. Rev. Neurobiol., 1987, vol. 3, pp 245-99
- Antoine J.C. et al., Journal of the Neurological Sciences, 1993, vol. 117, pp 215-223
- Byk et al., Journal of Neuroscience, 1996, vol. 16(2), pp 688-701
- Byk T, Ozon S, Sobel A (1998). Eur J. Biochem, 254:14-24
- Dalmau et al., Neurology, 1991, vol. 41, pp 1757-64
- Graus et al., Neurology, 1985, vol. 35, pp 538-543
- Greenlee et al., Ann. Neurol., 1983, vol. 14, pp 609-13
- Hamajima N, Matsuda K, Sakata S, Tamaki M, Nonaka M (1996). Gene, 180 :157-163.
- Hetzel et al., Mayo Clin. Proc., 1990, vol. 65, pp 1558-63
- Honnorat J. et al., Journal of Neurology, Neurosurgery and Psychiatry, 1996, vol. 61, pp 270-278
- Jaeckle et al., Ann. Neurol., 1985, vol. 18, pp 592-600
- Köhler, et Milstein, Nature, 1975, vol. 256, pp 495-497
- Levy N., Mattei MG., 1995, Geneprobs 11, a practical approch. BD Hames and SJ Higgins, Oxford University Press, pp 211-243
- Luque et al., Ann. Neurol., 1991, vol. 29, pp 241-51
- Sambrook et al., Molecular Cloning, a laboratory Manual, 1989, 9.47-9.62
- Wang LH and Strittmatter SM (1996). J Neurosci,16:6197-6207.

## Revendications

1. Polypeptide purifié désigné ULIP6, comprenant la séquence d'acides aminés. SEQ ID n° 2 ou fragment épitopique dudit polypeptide, comprenant la séquence SEQ ID n°4.

2. Acide nucléique isolé comprenant une séquence nucléotidique codant pour un polypeptide ou un fragment épitopique dudit polypeptide tel que défini dans la revendication 1.

3. Acide nucléique selon la revendication 2, comprenant une séquence nucléotidique SEQ ID n° 1.

4. Acide nucléique isolé consistant en une séquence nucléotidique SEQ ID n°3 ou la séquence allant du nucléotide 1 au nucléotide 162 de SEQ ID n° 1.

5. Vecteur de clonage et/ou d'expression contenant une séquence d'acide nucléique selon l'une des revendications 2 à 4.

6. Cellule hôte transfectée par un vecteur selon la revendication 5.

7. Anticorps mono- ou polyclonaux spécifiquement dirigés entre un polypeptide selon la revendication 1 ainsi que les fragments, les anticorps chimériques ou les immunoconjugués desdits anticorps mono- ou polyclonaux, lesdits fragments, anticorps chimériques ou immunoconjugués étant spécifiquement dirigés contre un polypeptide selon la revendication 1.

8. Composition utile pour le diagnostic des syndromes neurologiques paranéoplasiques et/ou pour le diagnostic précoce de la formation des tumeurs, **caractérisée en ce qu'**elle comprend un polypeptide ou un fragment épitopique dudit polypeptide tel que défini dans la revendication 1.

9. Utilisation d'un polypeptide selon la revendication 1 pour détecter la présence d'anticorps anti-CV2 dans un échantillon biologique.

10. Utilisation d'anticorps mono- ou polyclonaux ou leurs fragments, anticorps chimériques ou immunoconjugués selon la revendication 7, pour la purification ou la détection d'une protéine ULIP6 selon la revendication 1, dans un échantillon biologique.

11. Méthode pour le diagnostic des syndromes neurologiques paranéoplasiques et/ou pour le diagnostic précoce de la formation des tumeurs cancéreuses, dans lequel on met en évidence dans un échantillon biologique prélevé chez un individu des auto-anticorps dirigés contre une protéine ULIP 6 par
- la mise en contact d'un échantillon biologique prélevé chez un individu avec un polypeptide selon la revendication 1, éventuellement fixé sur un support dans des conditions permettant la formation de complexes immunologiques spécifiques entre ledit polypeptide et les auto-anticorps éventuellement présents dans l'échantillon biologique, et
- la détection des complexes immunologiques spécifiques éventuellement formés.

12. Kit pour le diagnostic des syndromes neurologiques paranéoplasiques et pour le diagnostic précoce de la formation des tumeurs à partir d'un prélèvement biologique comprenant :
- au moins un polypeptide ULIP6, selon: la revendication 1, éventuellement fixé sur un support,
- des moyens de révélation de la formation de complexes antigène/anticorps spécifiques entre un auto-anticorps anti-ULIP6 et ledit polypeptide ULIP6 et/ou des moyens de quantification de ces complexes.

13. Composition pharmaceutique comprenant au moins un agent thérapeutique choisi parmi un polypeptide selon la revendication 1, un acide nucléique selon la revendication 2 à 4 ou un acide nucléique comprenant une séquence anti-sens capable de s'hybrider spécifiquement avec un acide nucléique selon la revendication 2 à 4, ou un anticorps selon la revendication 7 spécifiquement dirigé contre le polypeptide, en association avec un véhicule pharmaceutiquement acceptable.

14. Utilisation d'un agent thérapeutique tel que défini dans la revendication 13 pour la fabrication d'un médicament destiné à traiter les maladies neurodégénératives et les néoplasmes.

## Claims

1. Purified polypeptide, termed ULIP6, comprising the amino acid sequence SEQ ID No. 2 or epitopic fragment of said polypeptide, comprising the sequence SEQ ID No. 4.

2. Isolated nucleic acid comprising a nucleotide sequence coding for a polypeptide or an epitopic fragment of said polypeptide as defined in claim 1.

3. Nucleic acid according to claim 2, comprising a nucleotide sequence SEQ ID No. 1.

4. Isolated nucleic acid comprising a nucleotide sequence SEQ ID No. 3 or the sequence running from nucleotide 1 to nucleotide 162 of SEQ ID No. 1.

5. Cloning and/or expression vector containing a nucleic acid sequence according to one of the claims 2 to 4.

6. Host cell transfected by a vector according to claim 5.

7. Mono- or polyclonal antibodies specifically directed against a polypeptide according to claim 1 and the fragments, the chimeric antibodies or the immunoconjugates of said mono- or polyclonal antibodies, said fragments, chimeric antibodies or immunoconjugates being specifically directed against a polypeptide according to claim 1.

8. Composition which is useful for diagnosis of paraneoplastic neurological syndromes and/or for early diagnosis of the formation of tumours, **characterised in that** it comprises a polypeptide or an epitopic fragment of said polypeptide as defined in claim 1.

9. Use of a polypeptide according to claim 1 for detecting the presence of anti-CV2 antibodies in a biological sample.

10. Use of mono- or polyclonal antibodies or their fragments, chimeric antibodies or immunoconjugates according to claim 7 for the purification or the detection of a ULIP6 protein according to claim 1, in a biological sample.

11. Method for diagnosis of paraneoplastic neurological syndromes and/or for early diagnosis of the formation of cancerous tumours in which, in a biological sample taken from an individual, auto-antibodies directed against a ULIP 6 protein are revealed by
- placing a biological sample taken from a individual in contact with a polypeptide according to claim 1, possibly fixed on a support in conditions which permit the formation of specific immunological complexes between said polypeptide and the auto-antibodies possibly present in the biological sample, and
- detection of the specific immunological complexes possibly formed.

12. Kit for diagnosis of paraneoplastic neurological syndromes and for early diagnosis of the formation of tumours from a biological sample comprising:
- at least one ULIP6 polypeptide according to claim 1, possibly fixed on a support,
- means for revealing the formation of specific antigen/antibody complexes between an anti-ULIP6 auto-antibody and said ULIP6 polypeptide and/or means for quantification of these complexes.

13. Pharmaceutical composition comprising at least one therapeutic agent chosen from a polypeptide according to claim 1, a nucleic acid according to claim 2 to 4 or a nucleic acid comprising an antisense sequence which is capable of hybridising specifically with a nucleic acid according to claim 2 to 4, or an antibody according to claim 7 specifically directed against the polypeptide, in association with a pharmaceutically acceptable vehicle.

14. Use of a therapeutic agent as defined in claim 13 for the production of a medicine which is intended for treating neurodegenerative diseases and neoplasms.

## Patentansprüche

1. Gereinigtes, als ULIP6 bezeichnetes Polypeptid, umfassend die Aminosäure-Sequenz SEQ ID Nr. 2 oder ein Epitop-Fragment dieses Polypeptids, welches die Sequenz SEQ ID Nr. 4 umfasst.

2. Isolierte Nukleinsäure, umfassend eine Nukleotid-Sequenz, die für ein Polypeptid oder ein Epitop-Fragment dieses Polypeptids, wie in Anspruch 1 definiert, codiert.

3. Nukleinsäure nach Anspruch 2, umfassend eine Nukleotid-Sequenz SEQ ID Nr. 1 .

4. Isolierte Nukleinsäure, die aus einer Nukleotid-Sequenz SEQ ID Nr. 3 oder der Sequenz besteht, die vom Nukleotid 1 bis zum Nukleotid 162 von SEQ ID Nr. 1 reicht.

5. Klonierungs- und/oder Expressionsvektor, enthaltend eine Nukleinsäure-Sequenz nach einem der Ansprüche 2 bis 4.

6. Wirtszelle, die mit einem Vektor nach Anspruch 5 transfiziert ist.

7. Mono- oder polyklonale Antikörper, die spezifisch gegen ein Polypeptid nach Anspruch 1 oder dessen Fragmente gerichtet sind, chimäre Antikörper oder Immunokonjugate dieser mono- oder polyklonalen Antikörper, wobei die Fragmente, chimären Antikörper oder immunokonjugierten Antikörper spezifisch gegen ein Polypeptid nach Anspruch 1 gerichtet sind.

8. Zusammensetzung, die für die Diagnose von paraneoplasischen neurologischen Syndromen und/oder für die frühzeitige Diagnose der Tumorbildung geeignet ist, **dadurch** charakterisiert, dass sie ein Polypeptid oder ein Epitop-Fragment dieses Polypeptids, wie in Anspruch 1 definiert, umfasst.

9. Verwendung eines Polypeptids nach Anspruch 1 zum Nachweis des Vorliegens von Anti-CV2-Antikörpern in einer biologischen Probe.

10. Verwendung von mono- oder polyklonalen Antikörpern oder ihren Fragmenten, chimären Antikörpern oder immunokonjugierten Antikörpern nach Anspruch 7 zur Reinigung oder zum Nachweis eines Proteins ULIP6 nach Anspruch 1 in einer biologischen Probe.

11. Verfahren zur Diagnose von paraneoplasischen neurologischen Syndromen und/oder zur frühzeitigen Diagnose der Bildung von Krebstumoren, bei dem man in einer biologischen Probe, die einem Individuum entnommen wurde, Auto-Antikörper nachweist, die gegen ein Protein ULIP6 gerichtet sind, durch
- In-Kontakt-Bringen einer einem Individuum entnommenen biologischen Probe mit einem Polypeptid nach Anspruch 1, gegebenenfalls auf einem Träger fixiert, unter Bedingungen, die die Bildung von spezifischen immunologischen Komplexen zwischen diesem Polypeptid und den AutoAntikörpern ermöglicht, die gegebenenfalls in der biologischen Probe vorhanden sind, und
- Nachweis der gegebenenfalls gebildeten spezifischen immunoloigschen Komplexe.

12. Kit zur Diagnose von paraneoplasischen neurologischen Syndromen und zur frühzeitigen Diagnose der Bildung von Tumoren, ausgehend von einer biologischen Probe, umfassend:
- mindestens ein Polypeptid ULIP6 nach Anspruch 1, gegebenenfalls auf einem Träger fixiert,
- Mittel zum Nachweis der Bildung von spezifischen Antigen/Antikörper-Komplexen zwischen einem Auto-Antikörper Anti-ULIPb und diesem Polypeptid ULIP6 und/oder Mittel zur quantitativen Bestimmung dieser Komplexe.

13. Pharmazeutische Zusammensetzung, umfassend mindestens ein therapeutisches Mittel, das ausgewählt wird aus einem Polypeptid nach Anspruch 1, einer Nukleinsäure nach Anspruch 2 bis 4 oder einer Nukleinsäure, umfassend eine Antisense-Sequenz, die zur spezifischen Hybridisierung mit einer Nukleinsäure nach Anspruch 2 bis 4 fähig ist, oder einem Antikörper nach Anspruch 7, der spezifisch gegen das Polypeptid gerichtet ist, in Verbindung mit einem pharmazeutisch verträglichen Vehikel.

14. Verwendung eines therapeutischen Mittels wie in Anspruch 13 definiert zur Herstellung eines Medikaments, das zur Behandlung von neurodegenerativen Erkrankungen und Neoplasmen bestimmt ist.


### LISTE DE SEQUENCES

<110> INSERM
<120> Nouvelle protéine ULIP/CRMP humaine et son utilisation dans le diagnostic et la thérapie des cancers et des syndromes neurologiques paranéoplasiques
<130> BET 01/0161
<140>
   <141>
<160> 4
<170> PatentIn Ver. 2.1
<210> 1
   <211> 3074
   <212> ADN
   <213> Homo sapiens
<220>
   <221> CDS
   <222> (163)..(1854)
<400> 1
   cccgccccac tctggactcc cgcgctgggc gcgctgaggc ggcccccgag cgagcgcgcg 60
   tgcagccgcc gccgccccga gcacccgcag ctccggcgcc gcggcgagac ggagacggac 120
   cgagccacgg gcccccgcgg ccgcagcatc tcggaggaga ac atg ctt gcc aac   174
                                                  Met Leu Ala Asn
                                                  1
   tca gcc agc gtg agg atc ctc atc aag gga ggc aag gtg gtg aac gat  222
   Ser Ala Ser Val Arg Ile Leu Ile Lys Gly Gly Lys Val Val Asn Asp
    5                  10                  15                  20
   gac tgc acc cac gag gct gac gtc tac atc gag aat ggc atc atc cag  270
   Asp Cys Thr His Glu Ala Asp Val Tyr Ile Glu Asn Gly Ile Ile Gln
                   25                  30                  35
   cag gtg ggc cgc gag ctc atg atc cct ggc ggc gcc aag gtg att gat  318
   Gln Val Gly Arg Glu Leu Met Ile Pro Gly Gly Ala Lys Val Ile Asp
               40                  45                  50
   gcc aca gga aaa ctg gtg atc cct ggt ggc atc gac acc agc acc cac  366
   Ala Thr Gly Lys Leu Val Ile Pro Gly Gly Ile Asp Thr Ser Thr His
           55                  60                  65
   ttc cac cag acc ttc atg aat gcc acg tgc gtg gac gac ttc tac cat  414
   Phe His Gln Thr Phe Met Asn Ala Thr Cys Val Asp Asp Phe Tyr His
       70                  75                  80
   ggg acc aag gca gca ctc gtc gga ggc acc acc atg atc atc ggc cac  462
   Gly Thr Lys Ala Ala Leu Val Gly Gly Thr Thr Met Ile Ile Gly His
   85                  90                  95                  100
   gtc ctg ccc gac aag gag acc tcc ctt gtg gac gct tat gag aag tgc  510
   Val Leu Pro Asp Lys Glu Thr Ser Leu Val Asp Ala Tyr Glu Lys Cys
                   105                 110                 115
   cga ggt ctg gcc gac ccc aag gtc tgc tgt gat tac gcc ctc cac gtg    558
   Arg Gly Leu Ala Asp Pro Lys Val Cys Cys Asp Tyr Ala Leu His Val
               120                 125                 130
   ggg atc acc tgg tgg gca ccc aag gtg aaa gca gaa atg gag aca ctg    606
   Gly Ile Thr Trp Trp Ala Pro Lys Val Lys Ala Glu Met Glu Thr Leu
           135                 140                 145
   gtg agg gag aag ggt gtc aac tcg ttc cag atg ttc atg acc tac aag    654
   Val Arg Glu Lys Gly Val Asn Ser Phe Gln Met Phe Met Thr Tyr Lys
       150                 155                 160
   gac ctg tac atg ctt cga gac agt gag ctg tac caa gtg ttg cac gct    702
   Asp Leu Tyr Met Leu Arg Asp Ser Glu Leu Tyr Gln Val Leu His Ala
   165                 170                 175                 180
   tgc aag gac att ggg gca atc gcc cgc gtc cat gct gaa aat ggg gag    750
   Cys Lys Asp Ile Gly Ala Ile Ala Arg Val His Ala Glu Asn Gly Glu
                   185                 190                 195
   ctt gtg gcc gag ggt gct aag gag gca ctg gat ttg ggg atc aca ggc    798
   Leu Val Ala Glu Gly Ala Lys Glu Ala Leu Asp Leu Gly Ile Thr Gly
               200                 205                 210
   cca gaa gga atc gag atc agc cgt cca gag gag ctg gaa gct gaa gcc    846
   Pro Glu Gly Ile Glu Ile Ser Arg Pro Glu Glu Leu Glu Ala Glu Ala
           215                 220                 225
   act cat cgt gtt atc acc att gca aac agg act cac tgt cca atc tac    894
   Thr His Arg Val Ile Thr Ile Ala Asn Arg Thr His Cys Pro Ile Tyr
       230                 235                 240
   ctg gtc aac gtg tcc agt atc tcg gct ggt gac gtt atc gca gct gct    942
   Leu Val Asn Val Ser Ser Ile Ser Ala Gly Asp Val Ile Ala Ala Ala
   245                 250                 255                 260
   aag atg caa ggg aag gtt gtg ctg gcg gag acc acc act gca cat gcc    990
   Lys Met Gln Gly Lys Val Val Leu Ala Glu Thr Thr Thr Ala His Ala
                   265                 270                 275
   acg ctc aca ggc tta cac tac tac cac cag gac tgg tcc cac gcg gct    1038
   Thr Leu Thr Gly Leu His Tyr Tyr His Gln Asp Trp Ser His Ala Ala
               280                 285                 290
   gcc tat gtc acg gtg cct ccc ctg aga ctg gac acc aac acc tca acc    1086
   Ala Tyr Val Thr Val Pro Pro Leu Arg Leu Asp Thr Asn Thr Ser Thr
           295                 300                 305
   tac ctc atg agc ctg ctg gcc aat gac act ctg aac atc gtg gca tca    1134
   Tyr Leu Met Ser Leu Leu Ala Asn Asp Thr Leu Asn Ile Val Ala Ser
       310                 315                 320
   gat cac cgg cct ttc acc aca aag cag aaa gct atg ggc aag gaa gac    1182
   Asp His Arg Pro Phe Thr Thr Lys Gln Lys Ala Met Gly Lys Glu Asp
   325                 330                 335                 340
   ttc acc aag atc cca cat gga gtg agt ggc gtg cag gac cgc atg agc    1230
   Phe Thr Lys Ile Pro His Gly Val Ser Gly Val Gln Asp Arg Met Ser
   345 350 355
   gtc atc tgg gag aga gga gtg gtt gga gga aag atg gat gag aac cgt 1278
   Val Ile Trp Glu Arg Gly Val Val Gly Gly Lys Met Asp Glu Asn Arg
   360 365 370
   ttt gtg gcc gtt acc agt tcc aac gca gct aag ctt ctg aac ctg tat 1326
   Phe Val Ala Val Thr Ser Ser Asn Ala Ala Lys Leu Leu Asn Leu Tyr
   375 380 385
   ccc cgc aag ggc cgc att att ccc gga gcc gat gct gat gtg gtg gtg 1374
   Pro Arg Lys Gly Arg Ile Ile Pro Gly Ala Asp Ala Asp Val Val Val
   390 395 400
   tgg gac cca gaa gcc aca aag acc atc tca gcc agc acg cag gtc cag 1422
   Trp Asp Pro Glu Ala Thr Lys Thr Ile Ser Ala Ser Thr Gln Val Gln
   405 410 415 420
   gga gga gac ttc aac ctg tat gag aac atg cgc tgc cac ggc gtg cca 1470
   Gly Gly Asp Phe Asn Leu Tyr Glu Asn Met Arg Cys His Gly Val Pro
   425 430 435
   ctg gtc acc atc agc cgg ggg cgc gtc gtg tat gag aac ggc gtc ttc 1518
   Leu Val Thr Ile Ser Arg Gly Arg Val Val Tyr Glu Asn Gly Val Phe
   440 445 450
   atg tgc gcc gag ggc acc ggc aag ttc tgt ccc ctg agg tcc ttc cca 1566
   Met Cys Ala Glu Gly Thr Gly Lys Phe Cys Pro Leu Arg Ser Phe Pro
   455 460 465
   gac act gtc tac aag aag ctg gtc cag aga gag aag act tta aag gtt 1614
   Asp Thr Val Tyr Lys Lys Leu Val Gln Arg Glu Lys Thr Leu Lys Val
   470 475 480
   aga gga gtg gac cgc act ccc tac ctg ggg gat gtc gct gtt gtc gtg 1662
   Arg Gly Val Asp Arg Thr Pro Tyr Leu Gly Asp Val Ala Val Val Val
   485 490 495 500
   cac cct ggg aaa aaa gag atg gga acc cca ctc gca gac act cct acc 1710
   His Pro Gly Lys Lys Glu Met Gly Thr Pro Leu Ala Asp Thr Pro Thr
   505 510 515
   cgg ccc gtc acc cgg cat ggc ggc atg agg gac ctt cac gaa tcc agc 1758
   Arg Pro Val Thr Arg His Gly Gly Met Arg Asp Leu His Glu Ser Ser
   520 525 530
   ttc agc ctc tct ggc tct cag atc gat gac cat gtt cca aag cga gct 1806
   Phe Ser Leu Ser Gly Ser Gln Ile Asp Asp His Val Pro Lys Arg Ala
   535 540 545
   tca gct cgg atc ctc gct cct ccc gga ggc agg tcg agt ggc att tgg 1854
   Ser Ala Arg Ile Leu Ala Pro Pro Gly Gly Arg Ser Ser Gly Ile Trp
   550 555 560
   taaaggcatt gccaagcccc ccgagtgagg acgcaccgcc gccaccagcc cgcaactctc 1914
   cagccgaagc tgcaggggca ggagaggctg ggctgggtgg cacaccaccc gagggggccc 1974
   ccgggaccca cggagccctc cctatgtctg caaagtgatt cactgtgctt cgagccaact 2034
   ctaacaggca ctttgagatg tgttcctcct gctgtagtcc tttctgcctt ggcctcggcg 2094
   ggcttttctg gggcccagga agcccacact atgcacagag cccaatgcat agagccctgg 2154
   ccagcccttc ctctcactcc tgcctccgct ggctttggga aagcccagac tttagtgccc 2214
   tgccccctgg ctgactggcc agttgcccag agcactttag cagatgtggt ttcaaagtaa 2274
   aggcctcctc ccccacccct taggccccgt ggtgacattt cccaagtcag acagatgtca 2334
   gcttcccagc catgcccagg acgtcctatc tcccccaacc cacctctggc cctgtgtagg 2394
   ggcagggatg ggggtggctg ggactcctgg tgcccctcgc cagcttctcc tgcgccccgc 2454
   ccacaccctc gggggggtca caggcccaga agggtagctg ggcggggctc gaggctggtg 2514
   ccaggcgcgt gtaaatggtt ttgttttgca cgtttggttt gcgcagtagt ttggtttgac 2574
   ttgtttgtgc atcctgtgaa aaataacggt gcttgtgtca ctagcataga atagcgacag 2634
   gaatagatgt ggtccttagg agacgctgca cttgacacca accagacagc acagggcagg 2694
   ggtggtggag ggggctgggc tcacaggcct ctcttttccc cgcctgcagt cttctgggct 2754
   gcgggaggcc ctggcccttt ccccttcccc tcccctcctt gtctagtttc ccacattcca 2814
   aaagggggcc tgggatgcta gccccagaga tgccagccct tcaggaagca ggtgtccttt 2874
   cccctctctg cccctgatca ctcccagcac tccccttgcc ttcccctgtc ttcacctgcc 2934
   accacacaca cacacacaca cacacacaca cacacacgca tggcttccta taacttcttc 2994
   ctgctggaca gagactcagc gctcctcctg tgtgactggc aagaggcctc atgcctgctg 3054
   agagagggtc gacgcggccg 3074
<210> 2
   <211> 564
   <212> PRT
   <213> Homo sapiens
<400> 2
   Met Leu Ala Asn Ser Ala Ser Val Arg Ile Leu Ile Lys Gly Gly Lys
    1               5                  10                  15
   Val Val Asn Asp Asp Cys Thr His Glu Ala Asp Val Tyr Ile Glu Asn
               20                  25                  30
   Gly Ile Ile Gln Gln Val Gly Arg Glu Leu Met Ile Pro Gly Gly Ala
           35                  40                  45
   Lys Val Ile Asp Ala Thr Gly Lys Leu Val Ile Pro Gly Gly Ile Asp
       50                  55                  60
   Thr Ser Thr His Phe His Gln Thr Phe Met Asn Ala Thr Cys Val Asp
   65                  70                  75                  80
   Asp Phe Tyr His Gly Thr Lys Ala Ala Leu Val Gly Gly Thr Thr Met
   85 90 95
   Ile Ile Gly His Val Leu Pro Asp Lys Glu Thr Ser Leu Val Asp Ala
   100 105 110
   Tyr Glu Lys Cys Arg Gly Leu Ala Asp Pro Lys Val Cys Cys Asp Tyr
   115 120 125
   Ala Leu His Val Gly Ile Thr Trp Trp Ala Pro Lys Val Lys Ala Glu
   130 135 140
   Met Glu Thr Leu Val Arg Glu Lys Gly Val Asn Ser Phe Gln Met Phe
   145 150 155 160
   Met Thr Tyr Lys Asp Leu Tyr Met Leu Arg Asp Ser Glu Leu Tyr Gln
   165 170 175
   Val Leu His Ala Cys Lys Asp Ile Gly Ala Ile Ala Arg Val His Ala
   180 185 190
   Glu Asn Gly Glu Leu Val Ala Glu Gly Ala Lys Glu Ala Leu Asp Leu
   195 200 205
   Gly Ile Thr Gly Pro Glu Gly Ile Glu Ile Ser Arg Pro Glu Glu Leu
   210 215 220
   Glu Ala Glu Ala Thr His Arg Val Ile Thr Ile Ala Asn Arg Thr His
   225 230 235 240
   Cys Pro Ile Tyr Leu Val Asn Val Ser Ser Ile Ser Ala Gly Asp Val
   245 250 255
   Ile Ala Ala Ala Lys Met Gln Gly Lys Val Val Leu Ala Glu Thr Thr
   260 265 270
   Thr Ala His Ala Thr Leu Thr Gly Leu His Tyr Tyr His Gln Asp Trp
   275 280 285
   Ser His Ala Ala Ala Tyr Val Thr Val Pro Pro Leu Arg Leu Asp Thr
   290 295 300
   Asn Thr Ser Thr Tyr Leu Met Ser Leu Leu Ala Asn Asp Thr Leu Asn
   305 310 315 320
   Ile Val Ala Ser Asp His Arg Pro Phe Thr Thr Lys Gln Lys Ala Met
   325 330 335
   Gly Lys Glu Asp Phe Thr Lys Ile Pro His Gly Val Ser Gly Val Gln
   340 345 350
   Asp Arg Met Ser Val Ile Trp Glu Arg Gly Val Val Gly Gly Lys Met
   355 360 365
   Asp Glu Asn Arg Phe Val Ala Val Thr Ser Ser Asn Ala Ala Lys Leu
   370 375 380
   Leu Asn Leu Tyr Pro Arg Lys Gly Arg Ile Ile Pro Gly Ala Asp Ala
   385 390 395 400
   Asp Val Val Val Trp Asp Pro Glu Ala Thr Lys Thr Ile Ser Ala Ser
   405 410 415
   Thr Gln Val Gln Gly Gly Asp Phe Asn Leu Tyr Glu Asn Met Arg Cys
   420 425 430
   His Gly Val Pro Leu Val Thr Ile Ser Arg Gly Arg Val Val Tyr Glu
   435 440 445
   Asn Gly Val Phe Met Cys Ala Glu Gly Thr Gly Lys Phe Cys Pro Leu
   450 455 460
   Arg Ser Phe Pro Asp Thr Val Tyr Lys Lys Leu Val Gln Arg Glu Lys
   465 470 475 480
   Thr Leu Lys Val Arg Gly Val Asp Arg Thr Pro Tyr Leu Gly Asp Val
   485 490 495
   Ala Val Val Val His Pro Gly Lys Lys Glu Met Gly Thr Pro Leu Ala
   500 505 510
   Asp Thr Pro Thr Arg Pro Val Thr Arg His Gly Gly Met Arg Asp Leu
   515 520 525
   His Glu Ser Ser Phe Ser Leu Ser Gly Ser Gln Ile Asp Asp His Val
   530 535 540
   Pro Lys Arg Ala Ser Ala Arg Ile Leu Ala Pro Pro Gly Gly Arg Ser
   545 550 555 560
   Ser Gly Ile Trp
<210> 3
   <211> 1218
   <212> ADN
   <213> Homo sapiens
<400> 3
   aggcattgcc aagccccccg agtgaggacg caccgccgcc accagcccgc aactctccag 60
   ccgaagctgc aggggcagga gaggctgggc tgggtggcac accacccgag gggggccccg 120
   ggacccacgg agccctccct atgtctgcaa agtgattcac tgtgcttcga gccaactcta 180
   acaggcactt tgagatgtgt tcctcctgct gtagtccttt ctgccttggc ctcggcgggc 240
   ttttctgggg cccaggaagc ccacactatc cacagagccc aatgcataga gccctggcca 300
   gcccttcctc tcactcctgc ctccgctggc tttgggaaag cccagacttt agtgccctgc 360
   cccctggctg actggccagt tgcccagagc actttagcag atgtggtttc aaagtaaagg 420
   cctcctcccc caccccttag gccccgtggt gacatttccc aagtcagaca gatgtcagct 480
   tcccagccat gcccaggacg tcctatctcc cccaacccac ctctggccct gtgtaggggc 540
   agggatgggg gtggctggga ctcctggtgc ccctcgccag cttctcctgc gccccgccca 600
   caccctcggg ggggtcacag gcccagaagg gtagctgggc ggggctcgag gctggtgcca 660
   ggcgcgtgta aatggttttg ttttgcacgt ttggtttgcg cagtagtttg gtttgacttg 720
   tttgtgcatc ctgtgaaaaa taacggtgct tgtgtcacta gcatagaata gcgacaggaa 780
   tagatgtggt ccttaggaga cgctgcactt gacaccaacc agacagcaca gggcaggggt 840
   ggtggagggg gctgggctca caggcctctc ttttccccgc ctgcagtctt ctgggctgcg 900
   ggaggccctg gccctttccc cttcccctcc cctccttgtc tagtttccca cattccaaaa 960
   gggggcctgg gatgctagcc ccagagatgc cagcccttca ggaagcaggt gtcctttccc 1020
   ctctctgccc ctgatcactc ccagcactcc ccttgccttc ccctgtcttc acctgccacc 1080
   acacacacac acacacacac acacacacac acacgcatgg cttcctataa cttcttcctg 1140
   ctggacagag actcagcgct cctcctgtgt gactggcaag aggcctcatg cctgctgaga 1200
   gagggtcgac gcggccgc                                               1218
<210> 4
   <211> 21
   <212> PRT
   <213> Homo sapiens
<400> 4
   Lys Glu Met Gly Thr Pro Leu Ala Asp Thr Pro Thr Arg Pro Val Thr
   1               5                   10                  15
   Arg His Gly Gly Cys
               20